(19) Europäisches Patentamt
European Patent Office
Office européen des brevets

(11) **EP 2 578 148 A1**

(12) **EUROPEAN PATENT APPLICATION**

(43) Date of publication:
**10.04.2013 Bulletin 2013/15**

(51) Int Cl.:
*A61B 5/055* (2006.01)   *A61B 5/107* (2006.01)
*A61B 5/113* (2006.01)   *G01R 33/565* (2006.01)

(21) Application number: **11183810.8**

(22) Date of filing: **04.10.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
• **Koninklijke Philips Electronics N.V.**
**5621 BA Eindhoven (NL)**
• **Philips Intellectual Property & Standards GmbH**
**20099 Hamburg (DE)**

(72) Inventors:
• **Forthmann, Peter**
**5600 AE Eindhoven (NL)**

• **Kuhn, Michael, H.**
**5600 AE Eindhoven (NL)**
• **Amthor, Thomas**
**5600 AE Eindhoven (NL)**
• **Krueger, Sascha**
**5600 AE Eindhoven (NL)**

(74) Representative: **Damen, Daniel Martijn**
**Philips**
**Intellectual Property & Standards**
**P.O. Box 220**
**5600 AE Eindhoven (NL)**

(54) **Medical imaging system with motion detection**

(57)    A medical imaging system comprises an image data acquisition module to acquire imaging data and a motion detection module to acquire motion information. A reconstruction module reconstructs image datasets from the imaging data and with use of the motion information to correct for motion. The motion detection module is provided with a shape-sensing photonic fibre system to provide a photonic output representative of the spatial shape of the photonic fibre and an arithmetic unit to compute the motion information on the basis of the photonic output.

Fig 5

**Description**

FIELD OF THE INVENTION

The invention pertains to medical imaging system comprising

- an image data acquisition module to acquire imaging data,
- a motion detection module to acquire motion information and
- a reconstruction module to reconstruct image datasets from the imaging data and with use of the motion information to correct for motion.

BACKGROUND OF THE INVENTION

Such a medical imaging system is known from the paper 'OFSETH: a breathing motions monitoring system for patients under MRI' by J. Dejonckheere et at. in the 32nd Annual International Conference of the IEEE EMBS. This known medical imaging system is an MRI system in which recording of respiratory movements is employed for correcting motion artefacts. A stretchable substrate such as an elastic textile fabric containing an optical fibre is employed for motion detection. When the substrate is stretched, the bending radius changes hence optical losses vary. The intensity variations at the output of the optical fibre then represent the relative variations of the substrate length. When light from a light source is coupled into the fibre, then at the output end by way of a light sensor, notably a photodiode, the frequency of the length variations of the substrate can be measured. In another embodiment an optical fibre Bragg grating (FBG) sensor is employed. The FBG sensor is sensitive to a strain applied to it. The applied strain induces a shift of the Bragg wavelength.

SUMMARY OF THE INVENTION

An object of the invention is to provide a medical imaging system with a motion detection module that is capable to acquire more comprehensive motion information.

This object is achieved by a medical imaging system comprising - an image data acquisition module to acquire imaging data

- a motion detection module to acquire motion information
- a reconstruction module to reconstruct image datasets from the imaging data and with use of the motion information to correct for motion, wherein
- the motion detection module is provided with

    i. a shape-sensing photonic fibre system to provide a photonic output representative of the spatial shape of the photonic fibre and

    ii. an arithmetic unit to compute the motion information on the basis of the photonic output.

An insight of the present invention is that the shape-sensing photonic fibre system's photonic fibre(s) can provide a time varying spatially three-dimensional encoding of the position and/or shape fibres. The encoding of the position and/or shape of the fibres is measured from the photonic output of the fibres. Form this encoding of the position and/or shape of the fibre the motion information relating to an object to be imaged can be derived. To this end, the output of the shape-sensing photonic fibre system is applied to the arithmetic unit which then computes the motion information from the time-varying position and/or shape of the fibres. Because the fibres are placed on or in the object to be imaged, variations of the position and/or shape of the fibres represent motion in or of the object to be imaged. Thus, the actual shape of the shape-sensing photonic fibre system is derived, which forms far more comprehensive information as compared to only the frequency of bending of an optical fibre or the applied strain. In an example of the shape-sensing optical fibre, the optical fibre is provided with a very large number (e.g. 10000 or more) FBG sensors that are spaced apart less than 1cm. The signals from these sensors are read-out using optical frequency domain reflectometry (OFDR) by which the very large number of sensors with the same nominal reflected wavelength to be read with a very high spatial resolution of e.g. less than $100\mu$m. Further details of the OFDR technique can be found in the paper High resolution optical frequency domain reflectometry for characterization of components and assemblies' by B.J. Soller in Optics Express 13, 666 (2005). Shape-sensing optical fibre are known per se from http://www.lunatechnologies.com/technology/shape-sensing.html and http://www.techbriefs.com/content/view/2585/3 6.

The photonic fibre system will not or only to a small extent affect the operation of the medical imaging system. Notably, when the medical imaging system is an magnetic resonance examination system, the operation of the magnetic resonance examination system is hardly affected. Namely, the shape-sensing photonic fibre system does not generate RF electromagnetic signals, at least not in the imaging region of the magnetic resonance examination system, Also, the shape-sensing photonic fibre system is not sensitive to the electro-magnetic fields, such as the RF ($B_1$) field, the gradient and main magnetic fields Further, the photonic fibre system has a high spatial and a high temporal resolution. The shape detected by the shape-sensitive photonic fibre system can be dynamically acquired at a rate of about 10shapes/s. From the dynamically acquired shapes during the entire diagnostic (e.g. MRI) examination patient motion can be detected and quantified and the diagnostic images can be corrected for patient motion. These corrections are useful in several clinical examinations. Head motion can be detected and corrected for in dynamic and functional magnetic resonance imaging. Thus, observed metabolic changes can be accurately allocated to the patient's anatomy. In multi-modality applications, such as MRI and CT or rotational X-ray imaging, accurately detected motion of the patient can be employed for accurate fusion of image data from the different modalities, e.g. magnetic resonance images can be accurately registered and fused with CT-images or rotational X-ray images. Accurate detection of tumour motion (e.g. due to respiration and cardiac motion) can be employed for accurate (MR) image guiding of radiotherapy and interventional oncology. Also the accurate motion detection supports precise percutaneous intervention. For example, an instrument such as a biopsy needle is accurately guided between the conductors of the RF coil array and the photonic fibres to reach the tumour.

Suitable photonic fibres for the shape-sensing photonic fibre system are fibres that transmit visible light, infrared or ultraviolet radiation.

The invention further achieves accurate motion correction when the shape-sensing photonic fibre system is employed in a computed-tomography (CT) system or in a rotational x-ray system. In CT imaging as well as in rotational x-ray and magnetic resonance imaging , the imaging data are acquired and subsequently the diagnostic image is reconstructed from the acquired imaging data. In magnetic resonance imaging the imaging data are formed by k-space profiles, in CT imaging and in rotational x-ray the imaging data are formed as x-ray absorption profiles from different orientations. Because the data acquisition time may be longer or comparable to the typical time-scale of movements in or of the object to be imaged, there may be a need for motion correction in the reconstruction of the diagnostic image. Because of the long data acquisition time, movement is likely to be significant during data acquisition so that motion artefacts may occur if they are not corrected for. The motion correction can be effected by correcting the imaging data for motion, or the motion correction can be performed in the reconstruction of the diagnostic image. These and other aspects of the invention will be further elaborated with reference to the embodiments defined in the dependent Claims.

According to one aspect of the invention, the shape-sensing photonic fibre system is linked to a fixed reference position. That is, one or more of the photonic fibres are e.g. at one of their ends fixed to one or respective reference position(s). These one or more reference positions form a basis for an absolute positioning of the three-dimensional encoding of the position and/or shape-sensing photonic fibres. The reference point can be any point of the shape sensing fibre and its position is measured in the same way as the position of all other points along the fibre. Knowing which point on the fibre is defined as the reference point and knowing the absolute position in space of this point (e.g. a fixed point on the patient support), the absolute positions of all other points on the fibre can be determined. Thus the three-dimensional encoding of the position and/or shape-sensing photonic fibres and its variations are determined relative to the fixed reference(s).

In another embodiment of the invention, from the three-dimensional encoding of the position and/or shape-sensing photonic fibres that is represented by the photonic output a time-varying surface shape in a three-dimensional geometry is calculated. This time-varying surface is then the basis for computation of the motion information.

In a further embodiment of the invention, the photonic fibres of the photonic shape-sensing fibre system are arranged to cover the object to be imaged in a spatially uniform manner. That is, the density of the coverage of the (surface of) the object is substantially equal over the portion of the surface. Consequently, the encoding has equal sampling density along the photonic fibres, and thus the surface shape is computed with uniform density. Notably, the uniform coverage ensures that the motion information is acquired at equal accuracy and at equal spatial resolution over the portion of the object covered.

According to a further implementation of the invention, the photonic output and the computation of the time-varying surface shape is done in real-time. This is achieved in that the read-out time from the shape-sensing photonic fibre system is much less than 1s, i.e. much less than the time-scale on which e.g. body motion of a patient to be examined, such as e.g. respiratory motion, occurs Thus, the motion correction can be performed over the entire acquired set of imaging data. Thus, no gating strategies are needed and hence less time is needed to image the object. In the particular, when the object is a patient to be examined, then reducing the required time for data acquisition improves clinical workflow and patient throughput.

According to a further aspect of the invention the medical imaging system is a magnetic resonance examination system and the shape-sensing photonic fibre system is provided in or on one or more of the RF transmit/receive antennae of a magnetic resonance examination system. Preferably, the photonic fibres are arranged along the electrical conductors of the RF transmit/receive antennae. In this implementation the photonic output from the shape-sensing photonic fibre system represents the spatial shape of the RF transmit/receive antennae. The shape-sensing photonic fibre system is able to monitor its shape over a length of several meters, up to 3m. The bending radius may be as small as 5mm and fibre diameters down to about $150 \mu m$ can be used. This allows most of the electrical conductors of an RF coil array to be monitored by a single photonic fibre.

On the basis of the spatial shape of the RF transmit/receive antenna, the spatial sensitivity profiles for receiving or transmitting of RF fields can be computed, notably with the use of Biot and Savarts law. Notably, the spatial distribution of the electrical conductors of the RF transmit/receive antenna determine the spatial sensitivity profiles for transmission and receiving of RF fields. Because the photonic output of also provides the temporal variations of the spatial shape the RF transmit/receive antennae, the arithmetic unit is able to provide regular updates of the spatial transmit/receive profiles of the RF antennae. On the basis of the spatial transmit/receive profiles of the RF transmit/receive antenna the image quality of the reconstructed magnetic resonance images can be improved. For example, brightness variations that are due to the spatial transmit/receive profile of the RF transmit/receive antennae can be corrected for. Further, in parallel imaging techniques, such as SENSE and GRAPPA etc. the acquired magnetic resonance signals are undersampled in k-space. Owing to the reduced sampling density in k-space, less than required by the Nyquist criterion for the field of view at issue, aliasing occurs in that the magnetic resonance signals from a pixel or voxel at issue contains a superposition of magnetic resonance signals from pixels/voxels that are spaced apart by the reduced field of view having the size of the reciprocal of the sampling distance on k-space. In parallel imaging, the contributions from several pixels/voxels are decomposed (unfolded) on the basis of the spatial receive profiles. According to the present invention, accurate spatial receive profiles are computed on the basis of the spatial shape of the (conductors of) the RF transmit/receive antennae. Because the spatial receive profiles are accurately measured by the shape-sensing photonic fibre system, accurate unfolding of the acquired data is achieved and only a low level of residual folding artefacts remain in the reconstructed magnetic resonance images. Moreover, because temporal variation of the spatial receive profiles is included, changes due to motion of the object being examined in the spatial receive profiles are accounted for in the parallel imaging reconstruction. Such change of the spatial receive profiles occur when the RF receive antennae, e.g. in the form of RF surface coil (arrays), are placed on the body of the patient to be examined and move e.g. due to respiratory motion of the patient.

Further, parallel excitation techniques, such as 'Transmit SENSE' by U. Katscher et al., as disclosed in MRM 49(2003)144-150 involve spatial intricate excitation profiles, such as a pencil beam excitation for a navigator, make use of the spatial transmit profiles of several independently driven RF transmit antennae. Notably, different components of the RF transmit field are provided by different RF transmit antennae. The actual transmitted field profile is determined by the activation level and the spatial transmit profile of the RF transmit antenna. Because the shape-sensing photonic fibre system accurately determines the spatial shape of the RF transmit antennae and accounts for temporal changes, accurate parallel excitation is enabled.

In another aspect of the intention the RF receive antenna is an RF surface coil that is placed on the body of the patient to be examined. The RF surface coil then follows the motion, notably respiratory motion, of the patient to be examined and may also be slightly deformed due to the patient's motion. Because the shape-sensing photonic fibres are mounted in the RF surface coil, in particular along the electrically conducting coil conductors (loop(s)) that acquire the magnetic resonance signals by picking-up flux, the photonic fibres follow the motion of the patient's body. Hence, from the varying surface shape computed from the photonic output, motion information of the patient's motion can be derived. That is, in this aspect of the invention, both the motion information of the patient's body as well as the actual spatial sensitivity profile of the RF surface coil can be computed from the varying surface shape.

These and other aspects of the invention will be elucidated with reference to the embodiments described hereinafter and with reference to the accompanying drawing wherein

BRIEF DESCRIPTION OF THE DRAWINGS

Fig. 1 shows a 'Front end' of an example of the shape-sensing photonic fibre system;
Fig. 2 shows the shape-sensing photonic fibre system of Figure 1 for a patient to be examined. The displacement and warping states of the fibres reflect the patient hull's topography.
Fig. 3 shows an example of the shape-sensing photonic fibre system that employs a fixed reference position
Fig. 4 shows a realisation diagram how to put into practice an RF coil array provided with a shape-sensitive photonic fibre system in an magnetic resonance examination system.
Fig. 5 shows a schematic representation of an magnetic resonance examination system in which the shape-sensing photonic fibre system of Figure 3 or 4 is employed.

DETAILED DESCRIPTION OF THE EMBODIMENTS

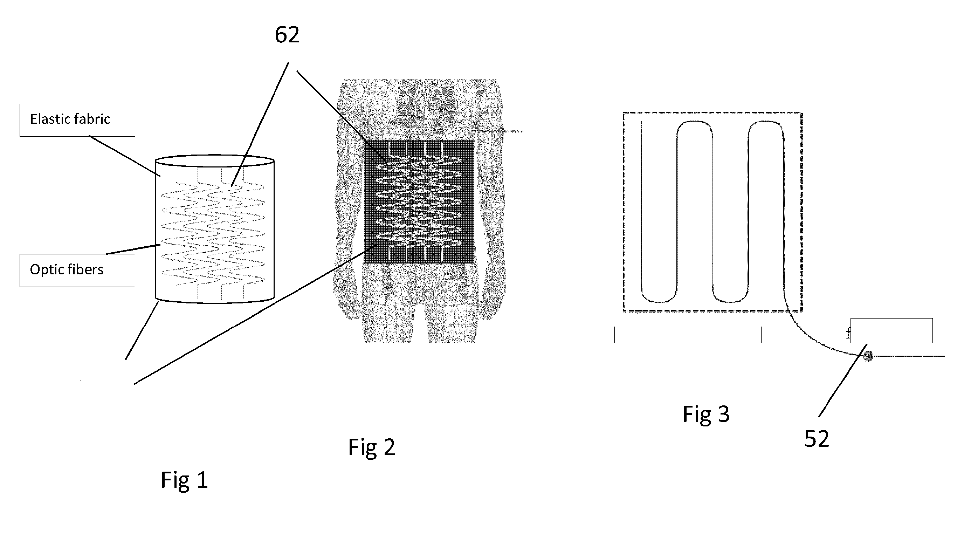

Figure 1 shows a 'Front end' of an example of the shape-sensing photonic fibre system. On a substrate in the form of an elastic fabric 60 there are disposed several shape-sensing photonic fibres 62. The photonic fibres may be optical fibres or (far) infrared fibres that have a good sensitivity for deformations of the fibres as well as an adequate transmission of photonic radiation, light or (far) infrared to the output end of the shape-sensing photonic fibre system. The light output from the shape-sensitive photonic fibre system represents the geometric shape of the fibres and its temporal changes. Because the fibres are disposed on or in the elastic fabric, the geometric shape of the fibres is directly related to the shape of the fabric.

Figure 2 shows the shape-sensing photonic fibre system for a patient to be examined. The displacement and warping states of the fibres reflect the patient hull's topography. In particular, Figure 2 shows the shape-sensing photonic fibre system implemented in a garment or wrapping structure that is placed over the patient to be examined such that the elastic fabric and consequently the incorporated photonic fibres 62 follows the movement and/or deformation of the patient's body, e.g. due to respiration and/or heart beat.

Figure 3 shows an example of the shape-sensing photonic fibre system that employs a fixed reference position. In this example, the shape-sensing photonic fibre system includes an optical fibre that is wound back and forth over the surface of the substrate 61. One end of the optical fibre is attached to a fixed point e.g. on the patient carrier of the medical imaging system. In this embodiment, from the output of the shape-sensitive photonic fibre system a uniform motion can be derived of the entire optical fibre on the substrate. Using the fixed reference point the absolute position can be measured of the patient surface in space. This can be used to determine the height of the chest's surface above the table, but also to detect sideways motion or tilting and rotation of the body. This information can for example be used for real-time control of different procedures, e.g. external beam radiation therapy. If the whole body of the patient moves or turns around, the radiation procedure could either be stopped or adapted automatically. The substrate may be provided with openings through which an interventional instrument, like a biopsy needle or catheter, can be passed into the patient's body. During an image guided intervention the shape-sensitive photonic fibre system may remain in place on the patient's body e.g. as depicted in Figure 2. Then, the motion information can be employed to correct the images that are acquired during the intervention for motion of the patient's body. This enables better image quality, less motion artefacts, in the images on which the interventional procedure is guided.

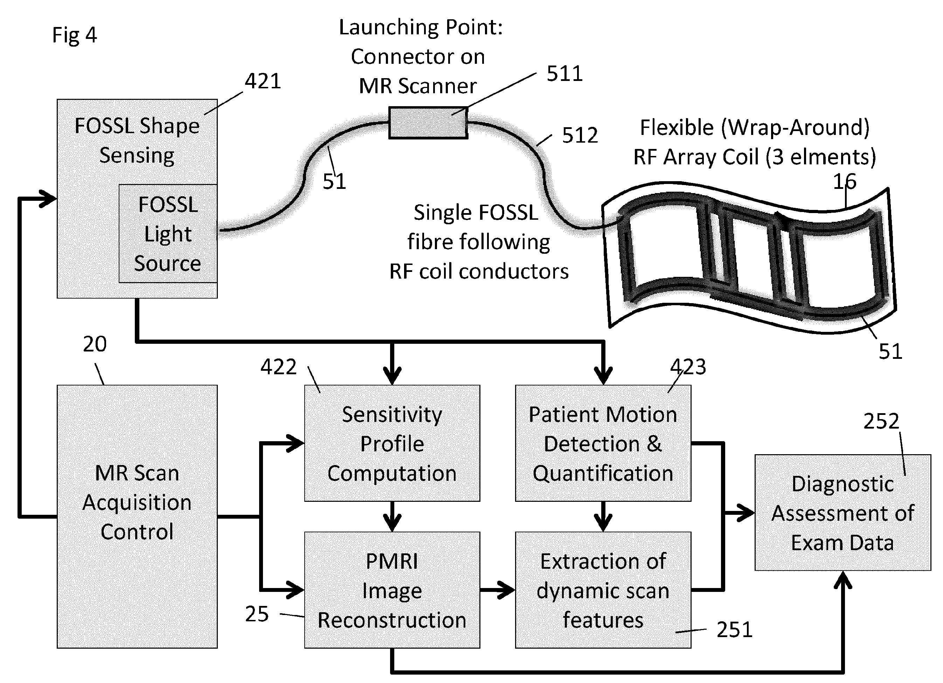

Figure 4 shows a realisation diagram how to put into practice an RF coil array provided with the shape-sensitive photonic fibre system 51 in an magnetic resonance examination system. The realization diagram shows the fibre-optic shape-sensing and localisation (FOSSL) shape-sensing module 421 which controls the operation of the shape-sensitive photonic fibre system 51. A light source 511 is provided , which brings the light to a launching point 52, which is rigidly defined in the MR system's coordinate system as a point in a connector attached to the patient table 14. The actual shape-sensing photonic fibre 512 is connected to this launching point 511 and follows the geometry of the conductors in the array coil. For example, a single fibre covers the surface and is connected to the readout module on one side. The reference point is just and arbitrary point on this one single fibre. The position of every point of the fibre is measured in the same way, no matter if the points are on the patient surface or on the part of the fibre leading to the connector.

The shape is computed from the light output from the output fibre 51. The light that returns at the output fibre represent the actual shape and position of the fibres disposed on or near the electrical conductor loops of the RF coil array 16. This shape is computed by the FOSSL shape-sensing module 421 and is then fed into a sensitivity profile computation unit 422 (potentially after being stored in a permanent storage medium for retrospective analysis), where the sensitivity profiles of the individual array coils are computed, e.g. via Biot and Savart's Law or the more complex software available from RF coil design. Then the profiles are fed into the parallel imaging reconstruction 25, where they allow to reconstruct artefact-free images.

In addition, the shape information is fed into the patient motion detection and quantification unit 423 (or fetched from that unit from the permanent storage medium), where the sequence of shapes detected over time is processed to detect and quantify patient motion, using suitable algorithms.

The motion information is then used to correct for motion in the extraction of dynamic scan features, e.g. in functional MRI or in dynamic contrast-enhanced MRI studies. These dynamic scan features are derived from the reconstructed magnetic resonance image by a feature extraction module 251. The extracted image features are corrected by a diagnostic assessment module 251 on the basis of the computed motion data from the patient motion detection and quantification 423. Both, the extracted features and the patient motion information is then used in the diagnostic assessment of the MRI examination data, via suitable visualization to the clinician and through further processing, potentially in a Computer-Aided Diagnosis (CAD) or a Clinical Decision Support (CDS) system.

In practice, the various modules, such as the FOSSL shape sensing module, sensitivity profile unit, motion detection and quantification unit, the feature extraction module, the diagnostic assessment module are implemented as software modules that communicated with the reconstructor 25 or may be implemented as software modules in reconstruction software of the reconstructor. These software modules are controlled by the controller function of the host computer 20. Alternatively, the various modules may be implemented in specially dedicated hardware, such as programmed integrated circuits.

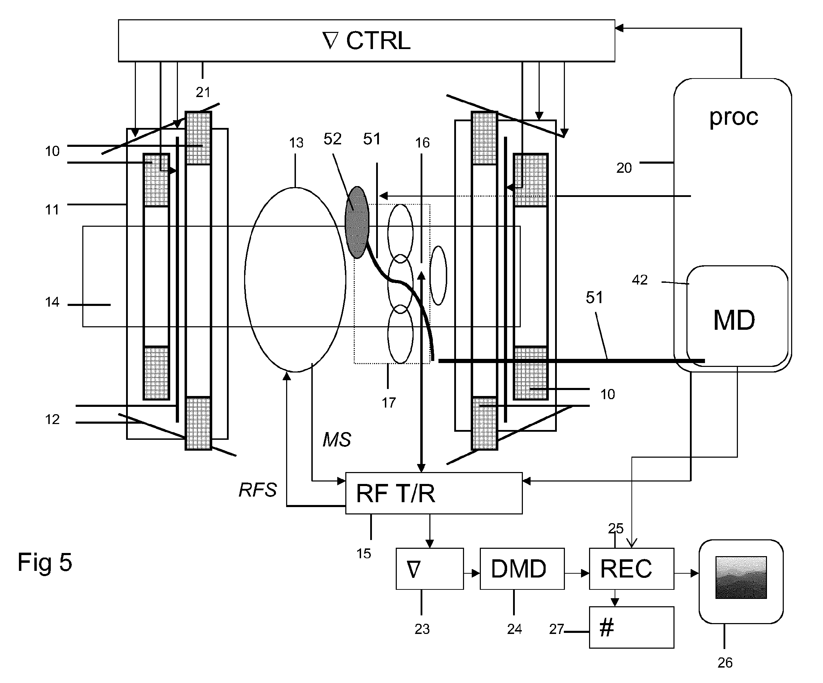

Figure 5 shows a schematic representation of an magnetic resonance examination system in which the shape-sensing photonic fibre system of Figure 3 or 4 is employed. The magnetic resonance imaging system includes a set of main coils 10 whereby the steady, uniform magnetic field is generated. The main coils are constructed, for example in such a manner that they enclose a tunnel-shaped examination space. The patient to be examined is placed on a patient carrier which is slid into this tunnel-shaped examination space. The magnetic resonance imaging system also includes a number of gradient coils 11, 12 whereby magnetic fields exhibiting spatial variations, notably in the form of temporary gradients in individual directions, are generated so as to be superposed on the uniform magnetic field. The gradient coils 11, 12 are connected to a gradient control 21which includes one or more gradient amplifier and a controllable power supply unit. The gradient coils 11, 12 are energised by application of an electric current by means of the power supply unit 21; to this end the power supply unit is fitted with electronic gradient amplification circuit that applies the electric current to the gradient coils so as to generate gradient pulses (also termed 'gradient waveforms') of appropriate temporal shape The strength, direction and duration of the gradients are controlled by control of the power supply unit. The magnetic resonance imaging system also includes transmission and receiving coils 13, 16 for generating the RF excitation pulses and for picking up the magnetic resonance signals, respectively. The transmission coil 13 is preferably constructed as a body coil 13 whereby (a part of) the object to be examined can be enclosed. The body coil is usually arranged in the magnetic resonance imaging system in such a manner that the patient 30 to be examined is enclosed by the body coil 13 when he or she is arranged in the magnetic resonance imaging system. The body coil 13 acts as a transmission antenna for the transmission of the RF excitation pulses and RF refocusing pulses. Preferably, the body coil 13 involves a spatially uniform intensity distribution of the transmitted RF pulses (RFS). The same coil or antenna is usually used alternately as the transmission coil and the receiving coil. Furthermore, the transmission and receiving coil is usually shaped as a coil, but other geometries where the transmission and receiving coil acts as a transmission and receiving antenna for RF electromagnetic signals are also feasible. The transmission and receiving coil 13 is connected to an electronic transmission and receiving circuit 15.

It is to be noted that it is alternatively possible to use separate receiving and/or transmission coils 16. For example, surface coils 16 can be used as receiving and/or transmission coils. Such surface coils have a high sensitivity in a comparatively small volume. The receiving coils, such as the surface coils, are connected to a demodulator 24 and the received magnetic resonance signals (MS) are demodulated by means of the demodulator 24. The demodulated magnetic resonance signals (DMS) are applied to a reconstruction unit. The receiving coil is connected to a preamplifier 23. The preamplifier 23 amplifies the RF resonance signal (MS) received by the receiving coil 16 and the amplified RF resonance signal is applied to a demodulator 24. The demodulator 24 demodulates the amplified RF resonance signal. The demodulated resonance signal contains the actual information concerning the local spin densities in the part of the object to be imaged. Furthermore, the transmission and receiving circuit 15 is connected to a modulator 22. The modulator 22 and the transmission and receiving circuit 15 activate the transmission coil 13 so as to transmit the RF excitation and refocusing pulses. In particular the surface receive coils 16 are coupled to the transmission and receive circuit by way of a wireless link. Magnetic resonance signal data received by the surface coils 16 are transmitted to the transmission and receiving circuit 15 and control signals (e.g. to tune and detune the surface coils) are sent to the surface coils over the wireless link.

The reconstruction unit derives one or more image signals from the demodulated magnetic resonance signals (DMS), which image signals represent the image information of the imaged part of the object to be examined. The reconstruction unit 25 in practice is constructed preferably as a digital image processing unit 25 which is programmed so as to derive from the demodulated magnetic resonance signals the image signals which represent the image information of the part of the object to be imaged. The signal on the output of the reconstruction monitor 26, so that the monitor can display the magnetic resonance image. It is alternatively possible to store the signal from the reconstruction unit 25 in a buffer unit 27 while awaiting further processing.

The magnetic resonance imaging system according to the invention is also provided with a control unit 20, for example in the form of a computer which includes a (micro)processor. The control unit 20 controls the execution of the RF excitations and the application of the temporary gradient fields. To this end, the computer program according to the invention is loaded, for example, into the control unit 20 and the reconstruction unit 25. The motion detection module 42 includes the shape-sensing photonic fibre system 51 that is integrated in the RF coil array 16. In this example, one of the photonic fibres of the shape-sensing photonic fibre system is fixed at the reference position 52 on the patient carrier 14. the shape-sensitive photonic fibre system 51 has its output fibre 53 coupled to the arithmetic unit 42 that is included in the processor 20. Notably, the arithmetic unit includes a photosensor which converts the photonic (e.g. light) output from the output fibre 53 into an electronic signal that is then digitally processed in the arithmetic unit 42 to compute the motion information. Further, the arithmetic unit is configured to compute the time-variations of the spatial sensitivity profiles of the RF coil array 16. The computations of the motion information and of the spatial sensitivity profiles are implemented in software modules in the arithmetic unit 42. The motion information is applied to the reconstructor 25 so that the reconstructor can account for motion of the patient to be examined in the reconstruction of the magnetic resonance image from the acquired magnetic resonance signals. The spatial sensitivity profiles of the RF coil array are applied to the reconstructor which employs these profiles in order to unfold aliasing due to undersampling of k-space in the acquisition of the magnetic resonance signals. Accurate parallel imaging is thus enabled which also accounts for variations in time of the spatial sensitivity profiles that may occur due to deformation and/or movement of the RF coil array. The arithmetic unit also computes the spatial emission profiles of the RF coil array. The spatial emission profiles may be computed from the output from the shape-sensing photonic fibre system, e.g.. based on the geometric configuration of the electrical conductors of the RF coil array using e.g. Biot and Savart's law

$$\mathbf{B} = \int \frac{\mu_0}{4\pi} \frac{I d\mathbf{l} \times \mathbf{r}}{|r|^3},$$

where
I is the electrical current,
dl is a vector, whose magnitude is the length of the differential_element of the wire, and whose direction is the direction of conventional current, B is the net magnetic field, $\mu 0$ is the magnetic constant, $\hat{\mathbf{r}}$ is the displacement unit vector in the direction pointing from the wire element towards the point at which the field is being computed, and $\mathbf{r} = r\hat{\mathbf{r}}$ is the full displacement vector from the wire element to the point at which the field is being computed. The symbols in boldface denote vector quantities. To apply the equation, you choose a point in space at which you want to compute the magnetic field. Holding that point fixed, you integrate over the path of the current (s) to find the total magnetic field at that point. The application of this law implicitly relies on the superposition principle for magnetic fields, i.e. the fact that the magnetic field is a vector sum of the field created by each infinitesimal section of the wire individually. For more details see http://en.wikipedia.org/wiki/Biot%E2%80%93Savart_law. Alternatively, based on reciprocity, the spatial emission profiles may be computed from the spatial sensitivity profiles. Because the shape-sensitivity photonic fibre system 51 has one of its fibres linked to the reference position 52 on the patient carrier, the arithmetic unit 42 can compute the surface shape of the fibres, and hence of the electrical conductors of the RF coil array in an absolute sense to the pre-determined position of the reference position 52

**Claims**

**1.** A medical imaging system comprising

    - an image data acquisition module to acquire imaging data

    - a motion detection module to acquire motion information
    - a reconstruction module to reconstruct image datasets from the imaging data and with use of the motion information to correct for motion, wherein
    - the motion detection module is provided with

        i. a shape-sensing photonic fibre system to provide a photonic output representative of the spatial shape of the photonic fibre and
        ii. an arithmetic unit to compute the motion information on the basis of the photonic output.

**2.** A medical imaging system as claimed in Claim 1, wherein the shape-sensing photonic fibre system is linked to a fixed reference position.

**3.** A medical imaging system as claimed in Claim 1, wherein the arithmetic unit is configured to compute a surface shape of the fibres varying in time and in a three-dimensional geometry and to compute the motion information from the time varying surface shape.

**4.** A medical imaging system as claimed in Claim 3, wherein the photonic fibre system's fibre spatially homogeneously covers an object to be imaged.

**5.** A medical imaging system as claimed in Claim 1, wherein the motion detection module is configured for real-time updating of the time varying surface shape.

**6.** A magnetic resonance examination system comprising

    - an image data acquisition module including one or more RF transmit and/or receive antenna (e) to acquire imaging data in the form of magnetic resonance signals
    - a reconstruction module to
    - reconstruct image datasets from magnetic resonance signals and
    - with use of the motion information to correct for motion, wherein
    - one or more RF transmit and/or receive antenna(e) is provided with

        i. a shape-sensing photonic fibre system to provide a photonic output representative of the spatial shape of the photonic fibre and
        ii. an arithmetic unit to compute the spatial receive and/or transmit profile of the one or more RF transmit and/or receive antenna (e) on the basis of the photonic output.

**7.** A magnetic resonance examination system as claimed in Claim 6, wherein the reconstruction module to reconstruct image datasets from the imaging data and is configured to correct for motion with use of the motion information, and

- the magnetic resonance examination system comprising a motion detection module to acquire motion information with

    i. the shape-sensing photonic fibre system to provide a photonic output representative of the spatial shape of the photonic fibre and an arithmetic unit to compute the motion information on the basis of the photonic output.

62

Elastic fabric

Optic fibers

Fig 1

Fig 2

Fig 3

52

Fig 4

Launching Point: Connector on MR Scanner — 511

FOSSL Shape Sensing — 421

FOSSL Light Source

Single FOSSL fibre following RF coil conductors — 51, 512

Flexible (Wrap-Around) RF Array Coil (3 elments) — 16, 51

MR Scan Acquisition Control — 20

Sensitivity Profile Computation — 422

Patient Motion Detection & Quantification — 423

PMRI Image Reconstruction — 25

Extraction of dynamic scan features — 251

Diagnostic Assessment of Exam Data — 252

EP 2 578 148 A1

Fig 5

**Europäisches Patentamt**
**European Patent Office**
**Office européen des brevets**

# EUROPEAN SEARCH REPORT

Application Number

EP 11 18 3810

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/054303 A1 (BARRICK EARL FREDERICK [US] ET AL) 3 March 2011 (2011-03-03) | 1-5 | INV. |
| Y | * paragraphs [0024], [0039] - [0041], [0044] - [0570], [0073] - [0079], [0083], [0084], [0087], [0088]; claims; figures * | 6,7 | A61B5/055 A61B5/107 A61B5/113 G01R33/565 |
| Y | US 6 668 184 B1 (KLEIMAN FELIX [IL]) 23 December 2003 (2003-12-23) * column 2, line 63 - column 5, line 45; claims; figures * | 6,7 | |
| Y | US 6 768 917 B1 (VAN VAALS JOHANNES JACOBUS [NL]) 27 July 2004 (2004-07-27) * column 4, lines 49-56 * * column 5, line 15 - column 7, line 23 * * column 8, lines 21-44; claims; figures * | 6,7 | |
| Y | US 5 947 900 A (DERBYSHIRE JOHN A [CA] ET AL) 7 September 1999 (1999-09-07) * column 2, lines 44-62 * * column 3, line 66 - column 10, line 12; claims; figures * | 6,7 | TECHNICAL FIELDS SEARCHED (IPC) A61B G01B G01R G01N G01D G01L G02B |
| Y | US 2008/204021 A1 (LEUSSLER CHRISTOPH G [DE] ET AL) 28 August 2008 (2008-08-28) * paragraphs [0021] - [0045]; claims; figures * | 6,7 | |
| Y | US 2008/081988 A1 (BIGLIERI EUGENIO [IT] ET AL) 3 April 2008 (2008-04-03) * paragraphs [0045], [0083] - [0127]; claims; figures * | 6,7 | |
| Y | SPIE, PO BOX 10 BELLINGHAM WA 98227-0010 USA, 2 July 2007 (2007-07-02), XP040242692, * the whole document * | 1-7 | |

-/--

The present search report has been drawn up for all claims

| Place of search | Date of completion of the search | Examiner |
|---|---|---|
| The Hague | 28 February 2012 | Crisan, Carmen-Clara |

Europäisches
Patentamt

European
Patent Office

Office européen
des brevets

# EUROPEAN SEARCH REPORT

Application Number

EP 11 18 3810

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PHILIP ST J RUSSELL: "Photonic-Crystal Fibers", JOURNAL OF LIGHTWAVE TECHNOLOGY, IEEE SERVICE CENTER, NEW YORK, NY, US, vol. 24, no. 12, 1 December 2006 (2006-12-01), pages 4729-4749, XP011156019, ISSN: 0733-8724, DOI: 10.1109/JLT.2006.885258 * page 4745 - page 4746 * ----- | 1-7 | |
| Y | MACPHERSON W N ET AL: "Remotely addressed optical fibre curvature sensor using multicore photonic crystal fibre", OPTICS COMMUNICATIONS, NORTH-HOLLAND PUBLISHING CO. AMSTERDAM, NL, vol. 193, no. 1-6, 15 June 2001 (2001-06-15), pages 97-104, XP027280936, ISSN: 0030-4018 [retrieved on 2001-06-15] * pages 97-98 * ----- | 1-7 | TECHNICAL FIELDS SEARCHED (IPC) |
| Y | BLANCHARD P M ET AL: "Two-dimensional bend sensing with a single, multi-core optical fibre", SMART MATERIALS AND STRUCTURES, IOP PUBLISHING LTD., BRISTOL, GB, vol. 9, no. 2, 1 April 2000 (2000-04-01), pages 132-140, XP020071338, ISSN: 0964-1726, DOI: 10.1088/0964-1726/9/2/302 * the whole document * ----- | 1-7 | |

The present search report has been drawn up for all claims

| Place of search | Date of completion of the search | Examiner |
|---|---|---|
| The Hague | 28 February 2012 | Crisan, Carmen-Clara |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**ANNEX TO THE EUROPEAN SEARCH REPORT
ON EUROPEAN PATENT APPLICATION NO.**                    EP 11 18 3810

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2012

| Patent document cited in search report | | Publication date | Patent family member(s) | | Publication date |
|---|---|---|---|---|---|
| US 2011054303 | A1 | 03-03-2011 | US 2002087101 A1<br>US 2011054303 A1 | | 04-07-2002<br>03-03-2011 |
| US 6668184 | B1 | 23-12-2003 | NONE | | |
| US 6768917 | B1 | 27-07-2004 | EP 1147430 A1<br>JP 2003511122 A<br>US 6768917 B1<br>WO 0125810 A1 | | 24-10-2001<br>25-03-2003<br>27-07-2004<br>12-04-2001 |
| US 5947900 | A | 07-09-1999 | NONE | | |
| US 2008204021 | A1 | 28-08-2008 | CN 1969195 A<br>EP 1761792 A2<br>US 2008204021 A1<br>WO 2005124380 A2 | | 23-05-2007<br>14-03-2007<br>28-08-2008<br>29-12-2005 |
| US 2008081988 | A1 | 03-04-2008 | AT 418738 T<br>EP 1906195 A1<br>EP 1978372 A2<br>ES 2318684 T3<br>US 2008081988 A1 | | 15-01-2009<br>02-04-2008<br>08-10-2008<br>01-05-2009<br>03-04-2008 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **J. DEJONCKHEERE.** OFSETH: a breathing motions monitoring system for patients under MRI. *32nd Annual International Conference of the IEEE EMBS*

- **B.J. SOLLER.** *Optics Express,* 2005, vol. 13, 666
- **U. KATSCHER et al.** Transmit SENSE. *MRM,* 2003, vol. 49, 144-150